Europäisches Patentamt

European Patent Office

Office européen des brevets

(19)

(11) Veröffentlichungsnummer: **0 345 246 B1**

(12) **EUROPÄISCHE PATENTSCHRIFT**

(45) Veröffentlichungstag der Patentschrift: **15.12.93**

(21) Anmeldenummer: **89890154.1**

(22) Anmeldetag: **22.05.89**

Die Akte enthält technische Angaben, die nach dem Eingang der Anmeldung eingereicht wurden und die nicht in dieser Patentschrift enthalten sind.

(51) Int. Cl.⁵: **A61L 25/00**

(54) **Gewebeklebstoff.**

(30) Priorität: **31.05.88 AT 1420/88**

(43) Veröffentlichungstag der Anmeldung:
**06.12.89 Patentblatt 89/49**

(45) Bekanntmachung des Hinweises auf die Patenterteilung:
**15.12.93 Patentblatt 93/50**

(84) Benannte Vertragsstaaten:
**AT BE CH DE ES FR GB IT LI LU NL SE**

(56) Entgegenhaltungen:
**EP-A- 0 253 198**

(73) Patentinhaber: **IMMUNO Aktiengesellschaft**
**Industriestrasse 67**
**A-1221 Wien(AT)**

(72) Erfinder: **Seelich, Thomas, Dr.**
**Gerhardusgasse 10/10**
**A-1200 Wien(AT)**

(74) Vertreter: **Wolfram, Gustav, Dipl.-Ing.**
**Patentanwälte**
**Sonn, Pawloy, Weinzinger & Wolfram,**
**Riemergasse 14**
**A-1010 Wien (AT)**

**Beschreibung**

Die Erfindung betrifft einen Gewebeklebstoff zum nahtlosen bzw. nahtunterstützenden Verbinden von menschlichen oder tierischen Gewebe- oder Organteilen, zur Wundversiegelung, Blutstillung und Förderung der Wundheilung in lyophilisierter Form, mit einem Gehalt an Fibrinogen von mindestens 0,25 (25 % Masse) und einem Gehalt an Faktor XIII, wobei das Mengenverhältnis des Faktors XIII zu Fibrinogen, ausgedrückt in Einheiten des Faktors XIII pro Gramm Fibrinogen, mindestens 150 beträgt.

Derartige Gewebeklebstoffe sind bereits aus der AT-B - 359.652 und AT-B - 369.990 bekannt. Sie enthalten neben Fibrinogen und Faktor XIII noch weitere Proteine, wie Fibronectin und Albumin und gegebenenfalls Antibiotika.

Ihre Wirkungsweise beruht darauf, daß durch Einwirkung von Thrombin das im rekonstituierten Gewebeklebstoff enthaltene (lösliche) Fibrinogen in (unlösliches) Fibrin übergeführt und der Faktor XIII zu Faktor XIIIa aktiviert wird. Dieser vernetzt das gebildete Fibrin zu einem Hochpolymer, was für die Wirksamkeit des Gewebeklebstoffes wesentlich ist. Die benötigte Thrombinaktivität kann entweder aus dem zu klebenden Gewebe (den Wundflächen) selbst stammen oder in Form einer Thrombin und $Ca^{2+}$-Ionen enthaltenden Lösung dem Gewebeklebstoff bei der Klebung zugesetzt werden.

Solche Präparate ermöglichen u.a. eine sichere Blutstillung, eine gute Haftfähigkeit des Klebstoffes an den Wund- bzw. Gewebsflächen, eine hohe Belastbarkeit der Klebungen bzw. Wundversiegelungen, eine vollkommene Resorbierbarkeit des Klebstoffes im Verlauf des Wundheilungsprozesses; und sie haben wundheilungsfördernde Eigenschaften.

Gewebeklebstoffe kommen entweder in Form tiefgefrorener Lösungen oder als Lyophilisat in den Handel, da sie als flüssige wässerige Lösungen wenig stabil und nicht für längere Zeit haltbar sind. Dieser Umstand führt dazu, daß die Handelsprodukte vor ihrer Applikation entweder aufgetaut oder aus ihrem Lyophilisat rekonstituiert werden müssen. Beide Maßnahmen sind mit einem nicht unerheblichen Zeitaufwand verbunden.

Ärztlicherseits besteht der Wunsch nach einer Verkürzung der Auflösungsdauer, da insbesondere in chirurgischen Notsituationen eine raschere Verfügbarkeit von entscheidender Bedeutung sein kann. Das Problem stellt sich besonders bei der Rekonstituierung eines Gewebeklebstofflyophilisates, weil für die Verwendung als Gewebeklebstoff Fibrinogen-Konzentrationen von mindestens 70 mg/ml erforderlich sind, die oft nur schwierig und zeitaufwendig erreicht werden können. Dies ist z.B. dann der Fall, wenn aus Gründen der biologischen Verträglichkeit der physiologische Salzgehalt der Lösung, bzw. die physiologische Ionenstärke, nicht überschritten werden darf (vgl. Redl et al., Medizinische Welt, 1985, 36, 769-76).

Eine besondere Schwierigkeit ergibt sich aus der Forderung nach erhöhter Sicherheit der Präparationen gegenüber dem Risiko der Übertragung pathogener Viren (Hepatitis, HIV), die im verwendeten Ausgangsmaterial (z.B. humanes Blutplasma) enthalten sein können; aus diesem Grund ist es notwendig, die Präparationen einem Virusinaktivierungsverfahren zu unterwerfen, das vorzugsweise derart durchgeführt wird, daß das pulverförmige Material bei geregeltem Feuchtigkeitsgehalt in geschlossenen Behältern während einer bestimmten Zeitdauer erhitzt wird. Allerdings verschlechtert sich dabei die Löslichkeit der Fibrinogenzubereitung weiter.

Dies gilt in noch höherem Maß bei Durchführung anderer Virusinaktivierungsverfahren, wie z.B. beim Erhitzen in Lösung in Gegenwart von Stabilisatoren.

Aus den dargelegten Gründen hat es nicht an Versuchen gefehlt, die Rekonstitutionszeit lyophilisierter Präparate zu verbessern. Die AT-B - 371.719 beschreibt beispielsweise ein Verfahren und eine Vorrichtung zur Beschleunigung des Auflösens lyophilisierter Arzneimittel. Das dort beschriebene kombinierte Wärme- und Rührgerät brachte zwar einen Fortschritt, d.h. deutlich verkürzte Rekonstitutionszeiten, doch werden ärztlicherseits weitere Verbesserungen gewünscht.

Es ist bekannt, daß die Löslichkeit von schwerlöslichen Proteinen durch bestimmte Zusätze verbessert werden kann. So beschreibt die EP-A - 0 085 923 eine lyophilisierte Fibrinogenzubereitung, die neben Fibrinogen noch eine Substanz enthält, die einen Harnstoff- oder einen Guanidinrest aufweist. Es hat sich allerdings gezeigt, daß derartige Zusätze zytotoxisch wirken, das Wachstum von Fibroblasten hemmen und zu einer veränderten, unphysiologischen Fibrinstruktur führen, wodurch die erwünschte Elastizität des Fibrins verlorengeht (vgl. Redl et al., loc. cit.). Mit der Wachstumshemmung der Fibroblasten, also derjenigen Zellen, die den Wundheilungsprozeß einleiten, gehen die erwünschten wundheilungsfördernden Eigenschaften von Gewebeklebstoffen auf Fibrinogenbasis verloren. Durch die fehlende Elastizität des entstandenen Fibrins wird weiters die geforderte hohe Belastbarkeit der Klebungen in vivo in Frage gestellt.

In der EP-A - 0 253 198 wird beschrieben, daß zur Verbesserung der Rekonstitution eines lyophilisierten Gewebeklebstoffes diesem vor der Lyophilisation Albumin und/oder Substanzen, die den Harnstoff oder Guanidinrest enthalten sowie gegebenenfalls eine Aminosäure mit hydrophober Seitenkette oder eine wasserlösliche Fettsäure und weiterhin Heparin zugesetzt werden. Obwohl Aminosäuren mit hydrophoben Seitenketten aufgrund ihrer Struktur zu den grenzflächenaktiven Verbindungen gerechnet werden können, hat sich herausgestellt, daß diese Aminosäuren, wie z.B. Isoleucin oder Leucin, nicht in der Lage sind, die Rekonstitutionszeit eines lyophilisierten Gewebeklebstoffes zu verkürzen.

Die vorliegende Erfindung stellt sich die Aufgabe, einen Gewebeklebstoff zur Verfügung zu stellen, der die erörterten Nachteile nicht aufweist und trotzdem eine gegenüber dem Stand der Technik wesentlich verkürzte Rekonstitutionszeit ermöglicht.

Diese Aufgabe wird erfindungsgemäß dadurch gelöst, daß der Gewebeklebstoff neben Fibrinogen und gegebenenfalls weiteren Proteinen sowie Hilfs- oder Zusatzstoffen mindestens ein Tensid aus den Gruppen der nichtionischen, kationischen, anionischen oder zwitterionischen Tenside in einer Menge von 0,0003 bis 0,15 (0,03 bis 15 % Masse), vorzugsweise 0,001 bis 0,01 (0,1 bis 1,0 % Masse), bezogen auf den Fibrinogen-Gehalt, enthält. Es wurde nämlich überraschenderweise gefunden, daß die Gegenwart dieser biologisch verträglichen Tenside die Rekonstitutionszeit wesentlich verkürzt, ohne die oben erwähnten nachteiligen Wirkungen zu zeigen.

Dies ist umso überraschender, als nach der gängigen Meinung der Fachwelt Tenside im allgemeinen eine denaturierende Wirkung auf Fibrinogen haben und sogar als Fällungsmittel für Fibrinogen verwendet wurden (vgl. Kurioka und Inove: "Interaction of Fibrinogen with Detergent", J. Biochem. 77, 449-455, 1975).

Insbesondere konnte gezeigt werden, daß Gewebeklebstoffe auf Fibrinogenbasis, die bestimmte Tenside in solchen Konzentrationen enthalten, die eine wesentliche Verkürzung der Rekonstitutionszeit des lyophilisierten Präparates bewirken, nicht zytotoxisch sind, das Wachstum von Fibroblasten nicht hemmen und die typische, räumlich verzweigte Struktur eines physiologischen Fibrinclots sowie dessen mechanische Eigenschaften (Reißfestigkeit und Elastizität) nicht verändern. Dies gilt für eine Vielzahl, in ihrem chemischen Aufbau völlig verschiedenartige Tenside aller vier Tensidklassen, also nichtionische, zwitterionische, anionische und kationische Tenside.

Die erfindungsgemäße Verwendung von Tensiden in Gewebeklebstoff-Präparationen ist in jedem Fall angezeigt, wenn die Präparationen im Verlauf ihrer Herstellung einem Virusinaktivierungsverfahren unterworfen werden, also auch dann sinnvoll, wenn die Präparationen zuletzt nicht durch Lyophilisieren, sondern durch Tieffrieren der gebrauchsfertigen Lösung haltbar gemacht werden sollen. Das ist z.B. dann der Fall, wenn in einer Zwischenstufe das Produkt lyophilisiert und einem Erhitzungsverfahren zum Zweck der Virusinaktivierung unterworfen wird. Das zugesetzte Tensid erleichtert die Wiederauflösung des erhitzten Zwischenproduktes und ermöglicht eine rasche Weiterverarbeitung.

Der Zusatz des Tensides kann daher in verschiedenen Stufen des Herstellungsprozesses erfolgen; insbesondere vor, wie auch nach Durchführung eines Virusinaktivierungsverfahrens. Dabei kann es zweckmäßig sein, daß der Gewebeklebstoff zur Stabilisierung noch zusätzlich ein Antioxidans enthält.

Der erfindungsgemäße Gewebeklebstoff ist mit aqua ad injectabilia zu einer gebrauchsfertigen Lösung mit einer Konzentration von mindestens 70 mg Fibrinogen/ml rekonstituierbar, welche Lösung eine Osmolarität von höchstens 0,70 Osmol aufweist, und ist sein Elektrolyt-Gehalt in der Weise begrenzt, daß nach einer weiteren zehnfachen Verdünnung mit aqua ad injectabilia die elektrische Leitfähigkeit bei 20 ° C höchstens 3 mS/cm beträgt.

Es ist bekannt, daß ein Gewebeklebstoff, dessen gebrauchsfertige Lösung eine unphysiologisch hohe Ionenstärke und/oder Osmolarität aufweist, zellschädigend wirkt.

Der Zusatz von Tensiden ermöglicht eine Verkürzung der Rekonstitutionszeit des erfindungsgemäßen lyophilisierten Gewebeklebstoffes, ohne die Ionenstärke und/oder Osmolarität der gebrauchsfertigen Lösung derart zu erhöhen, daß eine Zellschädigung beobachtet werden kann.

Als Maß des zulässigen Elektrolytgehaltes einer erfindungsgemäßen Präparation kann zweckmäßigerweise die elektrische Leitfähigkeit der daraus erhältlichen gebrauchsfertigen Lösung herangezogen werden, da diese auf einfache Weise exakt gemessen werden kann.

Der rekonstituierte erfindungsgemäße Gewebeklebstoff zeigt nach Vermischen mit einer Thrombin und $Ca^{2+}$-Ionen enthaltenden Lösung und Inkubation bei 37 ° C, eine vollständige Vernetzbarkeit der Fibrin-gamma-Ketten nach 3 bis 5 Minuten und eine mindestens 60 %ige Vernetzbarkeit der Fibrin-alpha-Ketten nach zwei Stunden, bestimmt mittels der Natriumlaurylsulfat-(SDS)-Polyacrylamid-Gelelektrophorese.

Es hat sich als günstig erwiesen, wenn der Gewebeklebstoff ein Tensid, ausgewählt aus den Gruppen der Polyätheralkohole, umfassend Polyoxyäthylen(23)dodecyläther, Polyoxyäthylen(10)hexadecyläther, Polyoxyäthylen(20)hexadecyläther, Octylphenolpolyäthylenglykol(30)äther, Octylphenolpolyäthylenglykol(12-13)äther, Octylphenolpolyäthylenglykol(7-8)äther, Octylphenolpolyäthylenglycol(40)äther und Octylphenolpolyäthylenglykolätherformaldehyd-polymere, der Polyätherester, wie Polyäthylenglykol-660-12-hydroxystearat, Polyoxyäthylen-stearoylester oder der Polyoxyäthylen-polyoxypropylen-Blockpolymeren, enthält.

3

Ebenso kann die Rekonstitutionszeit verkürzt werden, wenn der Gewebeklebstoff ein Tensid aus der Gruppe der Zuckerester, wie Sucrose-palmitat-stearat, der Polyalkoholanhydridester, wie Sorbitanmonolaurat oder Sorbitanmonooleat, der Glycoside, wie Octyl-$\beta$-D-glucopyranosid, der Alkinole, wie 2,4,7,9-Tetramethyl-5-decin-4,7-diol, der Aminoxide, wie Dodecyldimethylaminoxid, oder der Hydroxyalkylamide enthält.

Der gleiche positive Effekt kann bei einem Gewebeklebstoff beobachtet werden, der ein Tensid aus der Gruppe der Sulfosuccinate, wie Dioctylsulfosuccinat, oder der Alkalisalze der Gallensäuren, wie Na-Desoxycholat, enthält. Die genannten Verbindungen gehören der Klasse der anionischen Tenside an.

Des weiteren ist es vorteilhaft, wenn der Gewebeklebstoff ein Tensid aus der Gruppe der substituierten Ammoniumsalze, wie Benzyldimethyl-2-hydroxyäthylammoniumchlorid oder Benzyltrimethylammoniumchlorid, oder der Alkylpyridiniumsalze, wie Cetylpyridiniumchlorid, enthält. Diese Tenside sind kationischer Natur.

Auch die Gegenwart von zwitterionischen Tensiden verkürzt die Rekonstitutionszeit. Vorteilhaft kann ein erfindungsgemäßer Gewebeklebstoff ein Tensid enthalten aus der Gruppe der Phosphatide, wie Lecithin, oder ein Tensid aus der Gruppe der Sulfobetaine, wie N-Dodecyl-N'N-dimethylammonio-3-propansulfonat, der zwitterionischen Gallensäurenderivate, wie 3-(3-Cholamidopropyl)-dimethylamino-1-propansulfonat, der Alkylbetaine, der Sarkosine, wie N-Lauroylsarkosin, oder der Iminodipropionsäuren, wie N-Lauryl-$\beta$-iminodipropionsäure.

Zur Stabilisierung der erfindungsgemäßen Präparation, insbesondere bei Durchführung eines Virusinaktivierungsverfahrens, enthält sie gewünschtenfalls noch zusätzlich ein Antioxidans.

Als weitere vorteilhafte Ausführungsform kann der erfindungsgemäße Gewebeklebstoff zusätzlich Substanzen mit antimikrobieller Wirkung enthalten.

Die Erfindung umfaßt auch die Verwendung einer lyophilisierten Zusammensetzung auf Basis von menschlichen oder tierischen Proteinen mit einem Gehalt an Fibrinogen von mindestens 0,25 (25 % Masse) der Trockensubstanz, einem Gehalt an Faktor XIII in einer Menge von mindestens 150 Einheiten pro Gramm Fibrinogen und einem Gehalt an mindestens einem biologisch verträglichen Tensid, wobei der Gesamttensidgehalt 0,0003 bis 0,15 (0,03 bis 15 % Masse) des Fibrinogengehaltes beträgt, gegebenenfalls mit einem Gehalt an Fibronectin bis zu 0,65 (65 % Masse) des Fibrinogengehaltes und gegebenenfalls mit einem Gehalt an Albumin bis zu 1,1 (110 % Masse) des Fibrinogengehaltes, zur Herstellung einer gebrauchsfertigen Gewebeklebstofflösung in einer Konzentration von mindestens 70 mg Fibrinogen/ml zum nahtlosen bzw. nahtunterstützenden Verbinden von menschlichen oder tierischen Gewebe- oder Organteilen, zur Wundversiegelung, Blutstillung, Förderung der Wundheilung.

Die Erfindung wird nachfolgend noch näher erläutert.

A) Beispiele 1 bis 37

280 l humanes, bei -20°C eingefrorenes Frischplasma wurden auf +2°C erwärmt, das entstandene Kryopräzipitat durch Zentrifugieren abgetrennt, mit einer Pufferlösung vom pH-Wert 6,5, enthaltend 6,6 g Na$_3$-Citrat.2H$_2$O, 3,4g NaCl, 10,0 g Glycin, 25.000 KIE Aprotinin und 200 I.E. Heparin pro 1, behandelt und nochmals bei +2°C zentrifugiert. Der abgetrennte Niederschlag wurde in Portionen zu etwa 50 g geteilt und bis zur Weiterverarbeitung bei -20°C gelagert. Einzelne Portionen wurden sodann aufgetaut und in weiteren Pufferlösungen vom pH-Wert 7,9, enthaltend 19,0 g Humanalbumin, 9,0 g Glycin, 1,0 g Trinatriumcitrat.2H$_2$O, 25.000 KIE Aprotinin und 200 I.E. Heparin pro l, die außerdem noch verschiedene Tenside in unterschiedlichen Konzentrationen enthielten, gelöst und auf eine Proteinkonzentration von 50 g/l eingestellt. Die Bestimmung des Proteingehaltes erfolgt nach Kjeldahl.

Zur Herstellung einer Vergleichslösung (Kontrolle) wurde ein Teil des abgetrennten Niederschlages mit derselben Pufferlösung, jedoch ohne Tensidzusatz, wie oben beschrieben, gelöst und verdünnt.

Die verdünnten Gewebeklebstoff-Lösungen wurden sodann sterilfiltriert und zu je 2,5 ml in Endbehälter (Glasfläschchen) verfüllt, wobei ein Teil dieser Endbehälter mit einem magnetischen bzw. magnetisierbaren Rührkörper versehen wurde. Danach wurden sie in üblicher Weise tiefgefroren, gefriergetrocknet, die Endbehälter luftdicht verschlossen und bis zu den nachfolgend beschriebenen Tests bei +4°C gelagert.

Zur allgemeinen Charakterisierung der Präparationen wurden die folgenden Bestimmungen durchgeführt:
Trockensubstanz pro Endbehälter
Gesamtprotein (nach Kjeldahl)
Relativgehalt an Fibrinogen, Fibronectin und Albumin mittels SDS-Polyacrylamid-Gelelektrophorese in Gegenwart von Harnstoff (M. Furlan et al.: Plasmic degradation of human fibrinogen. IV. Identification of subunit chain remnants in fragment Y. Biochim. Biophys. Acta 400, 112-120 (1975), insbesondere Seite

4

114), u.zw. (a) einer nicht reduzierten und (b) einer mit $\beta$-Mercaptoäthanol reduzierten Probe, Färbung mit Coomassie-Blau und densitometrischer Auswertung (T. Seelich, H. Redl: Theoretische Grundlagen des Fibrinklebers, K. Schimpf: Fibrinogen, Fibrin und Fibrinkleber, F.K. Schattauer Verlag, Stuttgart-New York, 1980, Seiten 199-208).

Dabei wird in Probe (a) Albumin als isolierte Protein-Bande erhalten, woraus sich der Albumingehalt (in % vom Gesamtprotein) direkt ergibt. Probe (b) zeigt folgende isolierte Banden: Fibronectin, Fibrinogen -Aalpha-, -Bbeta- und gamma-Ketten, wobei zu beachten ist, daß in der Fibrinogen-Bbeta-Bande auch Albumin enthalten ist. Aus Probe (b) ergibt sich daher unmittelbar der Gehalt an Fibronectin; der Fibrinogengehalt ergibt sich aus der Summe der Fibrinogen-Aalpha,-Bbeta- und gamma-Banden nach Abzug des aus Probe (a) erhaltenen Albuminwertes.

Aus den so erhaltenen Werten für Fibrinogen, Fibronectin und Albumin (in % vom Gesamtprotein) und dem separat bestimmten Gesamtprotein kann somit der Gehalt an diesen drei Proteinen pro ml bzw. pro Endbehälter berechnet werden.

Der Gehalt an Fibrinogen wurde zusätzlich entsprechend der Vorschrift nach USP XVI, Seite 298, als das durch Thrombin gerinnbare Protein nach der Methode Kjeldahl bestimmt, wobei mit beiden Methoden gut übereinstimmende Werte erhalten wurden.

Zur Berechnung des Fibrinogengehaltes der Trockensubstanz wurde der nach der USP-Methode erhaltene Wert verwendet.

Der Gehalt an Faktor XIII wurde mittels eines Fibrin-Vernetzungstests, bei dem Faktor XIII-freies Fibrinogen als Substrat verwendet wird, auf folgende Weise bestimmt:

Je 0,5 ml einer Faktor XIII-freien Fibrinogenlösung mit einem Fibrinogengehalt von 10 mg/ml werden mit je 0,05 ml von verschiedenen Verdünnungen der zu bestimmenden Probe und je 0,1 ml einer Lösung, enthaltend 60 I.E. Thrombin und 130 $\mu$mol $CaCl_2$ pro ml, vermischt und bei 37°C inkubiert. Nach einer Inkubationszeit von 2 Stunden wird die Reaktion durch Zugabe eines Gemisches von Harnstoff, Na-Dodecylsulfat (SDS) und beta-Mercaptoäthanol gestoppt und werden die in den Proteinen enthaltenen Disulfidbrücken reduktiv gespalten. Der Vernetzungsgrad der Fibrin-gamma-Ketten wird nach SDS-Polyacrylamid-Gelelektrophorese (M. Furlan et al.: Plasmic degradation of human fibrinogen. IV. Identification of subunit chain remnants in fragment Y. Biochim. Biophys. Acta 400, 112-120 (1975), insbesondere Seite 114) der so erhaltenen Proben und Färbung mit Coomassie-Blau densitometrisch bestimmt und dient als Maß für den Faktor XIII-Gehalt.

Als Standard dient gepooltes humanes Citratplasma, wobei per definitionem 1 ml Plasma 1 Einheit Faktor XIII enthält. Diejenigen Verdünnungen der Probe und des Standards, die unter den Testbedingungen eine 50 %ige Vernetzung der Fibrin-gamma-Ketten bewirken, werden bestimmt, und der Faktor XIII-Gehalt (X) der ursprünglichen Probe nach der Formel

$$X = \frac{Vx}{Vs}$$

berechnet, wobei

Vx      die Verdünnung der unbekannten Probe und

Vs      die Verdünnung des Standards ist.

Aus dem so ermittelten Gehalt an Faktor XIII und dem wie oben bestimmten Fibrinogengehalt wurde das Verhältnis von Einheiten Faktor XIII zu Gramm Fibrinogen berechnet.

Elektrische Leitfähigkeit:

Bestimmung nach zehnfacher Verdünnung der gebrauchsfertigen Gewebeklebstoff-Lösung mit destilliertem Wasser unter Verwendung eines CDM-3-Konduktometers der Fa. Radiometer Copenhagen bei 20°C.

Osmolarität:

Bestimmung mit einem Dampfdruckosmometer der Fa. Wescor, USA.

Die Wirksamkeit und Unschädlichkeit der zugesetzten Tenside wurde folgendermaßen getestet:

Rekonstitutionszeit:

Es wurde jeweils jene Zeit in min gemessen, die zur vollständigen Auflösung des Lyophilisates nach Zugabe von 1,0 ml Lösungsmittel ($H_2O$ oder wässerige Aprotininlösung, 3000 KIE/ml) bei 37ºC benötigt wurde. Die zur Auflösung notwendige Durchmischung von Lyophilisat und Lösungsmittel erfolgte nach zwei Methoden: Fläschchen ohne magnetischem Rührkörper wurden von Hand leicht geschüttelt (Methode I) und bei Fläschchen mit Rührkörper wurde ein kombiniertes Rühr- und Wärmegerät verwendet. Dabei handelte es sich um ein spezielles, durch Induktion betreibbares und beheizbares Magnetrührgerät (Fibrinotherm[R], "Immuno" AG, Wien).

Die in Tabelle 1 angegebenen Rekonstitutionszeiten sind arithmetische Mittelwerte aus jeweils drei Bestimmungen.

Gehalt an gerinnbarem Protein (Fibrinogen): siehe oben.

Vernetzbarkeit der Fibrin-alpha-Ketten:

Die Bestimmung der Vernetzbarkeit der Fibrin-alpha-Ketten erfolgte nach einem Vernetzungstest (T. Seelich, H. Redl: "Theoretische Grundlagen des Fibrinklebers" in K. Schimpf: "Fibrinogen, Fibrin und Fibrinkleber", F.K. Schattauer Verlag, Stuttgart-New York, 199-208, 1980), bei dem nach Vermischen des gelösten, gebrauchsfertigen Gewebeklebstoffes mit dem gleichen Volumen einer Lösung, enthaltend 40 $\mu$mol $CaCl_2$ und 15 I.E. Thrombin pro ml, die Mischung bei 37°C inkubiert wird. Der Vernetzungsgrad der Fibrin-alpha-Ketten wird nach Stoppen der Reaktion und reduktiver Spaltung der in den Proteinen enthaltenen Disulfid-Brücken durch Zugabe eines Gemisches von Harnstoff, Na-Dodecylsulfat (SDS) und beta-Mercaptoäthanol mittels SDS-Polyacrylamid-Gelelektrophorese und Färbung mit Coomassie-Blau densitometrisch bestimmt.

Charakterisierung des gebildeten Fibrins:

Wie bereits in der Literatur beschrieben, ist die Art der gebildeten Fibrinstruktur bereits makroskopisch zu erkennen: die typische, räumlich verzweigte Struktur, die unter physiologischen Bedingungen entsteht, äußert sich makroskopisch in Form weißer, zäh-elastischer Clots (englisch: "coarse clots"). Unter unphysiologischen Bedingungen entstehen hingegen durchsichtige, spröde sogenannte "fine clots" (vgl. Ferry und Morrison, J. Am. Chem. Soc. 69, 388-400, 1947; Redl et al., Med. Welt 36, 769-776, 1985).

Zur Charakterisierung des gebildeten Fibrins wurden daher gleiche Volumina der zu testenden, gebrauchsfertigen Gewebeklebstofflösung und einer Thrombin-$CaCl_2$-Lösung (15 I.E. Thrombin und 40 $\mu$mol $CaCl_2$ pro ml) bei 37°C gemischt und nach etwa 10 min das Aussehen und die Konsistenz (Festigkeit, Elastizität) des entstandenen Fibrin-Clots beurteilt.

Zellverträglichkeit nach Redl et al., Medizinische Weit 36, 769-776, 1985:

Die zu testende Gewebeklebstofflösung wurde mit isotoner Kochsalzlösung 1 + 1 verdünnt und mit dieser Lösung lebende humane Lungenfibroblasten, die in Zellkultur bis zur Bildung eines gleichmäßigen Zellrasens gezüchtet worden waren, überschichtet. Eine etwaige schädigende Wirkung des Gewebeklebstoffes (bzw. des darin enthaltenen Tensids) auf die lebenden Zellen wurde im Lichtmikroskop beobachtet. Nach etwa 1 h Inkubationszeit wurden die Zellen zur genaueren Beurteilung fixiert und gefärbt.

In gesonderten Versuchen wurden Gewebeklebstofflösungen entsprechend dem Vergleich zu den Beispielen 1 bis 37 steigende Mengen an NaCl oder Saccharose zugesetzt, und mit diesen Lösungen der oben beschriebene Test auf Zellverträglichkeit durchgeführt. Diese Versuche dienten dazu, festzustellen, ab welchem Elektrolytgehalt bzw. ab welchem Osmolarität eine zellschädigende Wirkung beobachtet werden kann.

Es wurde festgestellt daß eine zellschädigende Wirkung ab einer Osmolarität von über 0,70 Osmol und/oder ab einer elektrischen Leitfähigkeit (der zehnfach mit Aqua ad iniectabilia verdünnten Lösung entsprechend dem oben beschriebenen Test) von über 3 mS/cm eintritt.

Die Beispiele 1 bis 37 sowie das dazugehörige Vergleichsbeispiel unterscheiden sich hinsichtlich ihrer Zusammensetzung lediglich durch ihren unterschiedlichen bzw. fehlenden Tensidgehalt. Der Fibrinogengehalt betrug 48 bis 51 % der Trockensubstanz, wobei diese geringen Unterschiede durch den unterschiedlichen Tensidgehalt bedingt sind.

Nach Rekonstitution des in einem Endbehälter enthaltenen Lyophilisates mit 1,0 ml $H_2O$ wurde eine Fibrinogenkonzentration von etwa 80 mg/ml erhalten. Der Relativgehalt an Fibrinogen wurde mit 64 %, an Fibronectin mit 4,7 % und an Albumin mit 26 % des Gesamtproteins bestimmt. Daraus ergibt sich ein Massenverhältnis von Fibronectin zu Fibrinogen von 0,07 und von Albumin zu Fibrinogen von 0,41.

Die elektrische Leitfähigkeit (nach weiterer zehnfacher Verdünnung mit $H_2O$) betrug bei allen Beispielen etwa 1,3 mS/cm bei 20°C, d.h. der relativ geringe Tensidzusatz hatte keinen merklichen Einfluß auf die elektrische Leitfähigkeit.

Ebenso wurde bei allen Beispielen eine Osmolarität (der konzentrierten Gewebeklebstofflösungen) von etwa 0,45 Osmol gefunden. Das Verhältnis von Einheiten Faktor XIII zu Gramm Fibrinogen betrug bei allen Beispielen 170.

Der unerwartet günstige Einfluß von Tensiden auf die Rekonstitutionszeit lyophilisierter Gewebeklebstoffpräparationen kann Tabelle 1 entnommen werden, in der weiters der Gehalt an gerinnbarem Protein und die Vernetzbarkeit der Fibrin-alpha-Ketten angegeben ist.

Mit den Nummern 1 bis 37 sind Beispiele für Gewebeklebstoffpräparationen bezeichnet, die verschiedene Tenside enthalten, deren genaue chemische Natur Tabelle 2 entnommen werden kann. Sie dokumentieren, daß eine Vielzahl von nichtionischen, zwitterionischen, anionischen und kationischen Tensiden geeignet ist, die Rekonstitutionszeit wesentlich zu verkürzen. Im Schnitt konnte das Lyophilisat durch den Tensidzusatz sowohl nach Methode I als auch nach Methode II etwa doppelt bis dreimal so schnell gelöst werden wie die Vergleichsprobe für die Beispiele 1 bis 37, dessen Rekonstitutionszeit 15 min nach Methode I betrug und 7 min bei Verwendung des kombinierten Rühr- und Wärmegerätes (Methode II).

Dabei wurde durch die zugesetzten Tenside die Gerinnbarkeit des Fibrinogens praktisch überhaupt nicht, und die Vernetzbarkeit der Fibrin-alpha-Ketten nur geringfügig beeinflußt.

Die vorteilhaften biochemischen, physikalischen und biologischen Eigenschaften der Präparate wurden durch den Tensidzusatz nicht beeinträchtigt. Sie erwiesen sich in allen Fällen als nicht-zytotoxisch und die sich bildenden Fibrin-Clots waren weiß und von erwünschter zähelastischer Konsistenz.

Die Beispiele 17, 21 und 37 zeigen, daß auch Kombinationen verschiedener Tenside verwendet werden können, um die Rekonstitutionszeit ohne unerwünschte Nebenwirkungen zu verkürzen.

Beispiel 37 zeigt insbesondere, daß selbst Kombinationen von anionischen und kationischen Tensiden zum gewünschten Ergebnis führen. Die beiden Tenside A2 und K1 wurden im äquimolaren Verhältnis eingesetzt, wodurch, ähnlich wie bei zwitterionischen Tensiden, die zugesetzten Tenside insgesamt die gleiche Anzahl an positiven und negativen Ladungen tragen.

Den Beispielen 31 bis 36 ist zu entnehmen, daß auch anionische oder kationische Tenside allein mit Erfolg eingesetzt werden können. Beispiel 23 zeigt die Verwendung eines nichtionischen Tensides (N17) aus der Gruppe der Polyätheralkohole, das zur Stabilisierung (Schutz vor Auto-Oxidation) zusätzlich noch ein Antioxidans enthält.

Die folgenden Beispiele 38 bis 42 zeigen, daß ein ebenso günstiger Effekt erzielt wird, wenn die Fibrinogenpräparation einem Virusinaktivierungsverfahren unterworfen wird.

Beispiel 38 und 38a:

Eine Gewebeklebstoffpräparation wurde auf ähnliche Weise hergestellt wie in den Beispielen 1 bis 37 beschrieben, jedoch wurden der verdünnten Lösung zehn Einheiten Faktor XIII pro ml zugegeben. Die Lösung wurde sodann im Ganzen tiefgefroren, lyophilisiert und bei einem Wassergehalt von 0,005 (0,5 % Masse) unter $N_2$-Atmosphäre 30 h lang auf 60°C erhitzt.

Danach wurde das erhitzte Lyophilisat in zwei Teile geteilt und einerseits mit $H_2O$ (Vergleich) und andererseits mit einer 0,01 Gew.%igen wässerigen Lösung des Tensides Pluronic F108 (siehe Tabelle 2) zu einer Proteinkonzentration von 50 g/l gelöst. Beide Lösungen wurden sterilfiltriert und zu je 2,5 ml in Endbehälter (Glasfläschchen) mit oder ohne Rührkörper verfüllt, tiefgefroren und lyophilisiert.

Nach Rekonstitution mit 1,0 ml $H_2O$ (Beispiel 38) bzw. wässeriger Aprotininlösung (Beispiel 38a) wurden gebrauchsfertige Gewebeklebstofflösungen mit etwa 80 mg Fibrinogen/ml erhalten. Die benötigten Rekonstitutionszeiten betrugen für die erfindungsgemäße, tensidhältige Präparation 7 min nach Methode I (Schütteln von Hand) und 3 bis 4 min nach Methode II (Verwendung des kombinierten Wärme- und Rührgerätes).

Bei der nicht tensidhältigen Vergleichspräparation betrugen die entsprechenden Zeiten 20 bzw. 9 min. Das Verhältnis von Faktor XIII zu Fibrinogen wurde mit etwa 420 Einheiten pro Gramm bestimmt.

Im übrigen unterschieden sich die beiden Präparationen nicht merklich voneinander und entsprachen in ihren sonstigen Eigenschaften den in den Beispielen 1 bis 37 beschriebenen Gewebeklebstoff-Präparationen.

Beispiel 39:

Die Herstellung verdünnter Gewebeklebstoff-Lösungen ohne Tensidgehalt (Teil I) und mit einem Gehalt an Triton WR 1339 (siehe Tabelle 2) in einer Konzentration von 0,1 g/l (Teil II) erfolgte wie in den Beispielen 1 bis 37 beschrieben, jedoch wurden den Lösungen zusätzlich 15.000 Einheiten Faktor XIII pro l zugesetzt.

Beide Lösungen wurden sodann im Ganzen lyophilisiert, auf einen Wassergehalt von 0,075 (7,5 Gew.%) eingestellt und unter $N_2$-Atmosphäre 10 h lang auf 60°C erhitzt. Danach wurden beide Teile zu einer Proteinkonzentration von 50 g/l gelöst. Die Auflösung von Teil I war langwierig und gelang nicht vollständig; Teil II ließ sich hingegen leicht und vollständig lösen. Beide Teile wurden zuerst klarfiltriert und dann sterilfiltriert, zu 5,0 ml in Endbehälter (Glasfläschchen) mit oder ohne magnetische Rührkörper verfüllt und in üblicher Weise tiefgefroren und lyophilisiert.

Nach Rekonstitution mit 2,0 ml $H_2O$ wurden gebrauchsfertige Gewebeklebstoff-Lösungen mit etwa 80 mg Fibrinogen/ml erhalten. Die benötigten Rekonstitutionszeiten betrugen für die erfindungsgemäße, tensidhältige Präparation 8 min nach Methode I (Schütteln von Hand) und 4 min nach Methode II (kombiniertes Wärme- und Rührgerät).

Für die nicht tensidhältige Vergleichspräparation betrugen die entsprechenden Zeiten 25 bzw. 12 min.

Das Verhältnis Faktor XIII zu Fibrinogen betrug etwa 520 E/g; in ihren übrigen Eigenschaften entsprachen die beiden Präparationen denen der vorhergehenden Beispiele.

Beispiel 40: (Hitzebehandelter Gewebeklebstoff mit einem Tensid- und Antibiotikumgehalt)

Die Herstellung tensidfreier bzw. tensidhältiger hitzebehandelter lyophilisierter Gewebeklebstoff-Präparationen erfolgte wie in dem vorhergehenden Beispiel 39 beschrieben, jedoch wurden den verdünnten Gewebeklebstofflösungen 20 Einheiten Faktor XIII pro ml zugesetzt und wurden die Lyophilisate mit einer Wässerigen Lösung, enthaltend 18 g Gentamycinsulfat pro 1, die mit NaOH auf pH 7,3 eingestellt worden war, gelöst.

Die Lösungen wurden sterilfiltriert, zu 12,5 ml in Endbehälter mit Rührkörper verfüllt und lyophilisiert.

Nach Rekonstitution mit 5,0 ml $H_2O$ oder wässeriger Aprotininlösung wurden Gewebeklebstofflösungen mit etwa 80 mg Fibrinogen/ml und einem Gentamycingehalt von 45 mg/ml erhalten.

Der Fibrinogengehalt der lyophilisierten Präparation betrug 38 % Masse, das Verhältnis Faktor XIII zu Fibrinogen etwa 750 Einheiten pro Gramm. Die elektrische Leitfähigkeit (nach zehnfacher Verdünnung mit $H_2O$) betrug 2,2 mS/cm, die Osmolarität der konzentrierten, gebrauchsfertigen Lösung etwa 0,61 Osmol,

Die benötigte Rekonstitutionszeit (Methode II) betrug für die erfindungsgemäße, tensidhaltige Präparation 4 min, für die tensidfreie, jedoch antibiotikumhaltige Vergleichspräparation jedoch 25 min.

Daraus geht hervor, daß in diesem Fall der Tensidgehalt der Präparation von besonders großem Vorteil ist, da die Auflösbarkeit der lyophilisierten Gewebeklebstoff-Präparation durch den Antibiotikumgehalt an sich verschlechtert wird, wie ein Vergleich mit einer tensid- und antibiotikumfreien Vergleichspräparation (Vergleich für 39) zeigt. Diese unerwünschte Wirkung des Antibiotikums wird durch den Tensidgehalt vollständig aufgehoben.

Beispiel 41 (Entfernung lipophiler Bestandteile durch Zusatz eines nichtionischen Tensides, Erwärmen über den Trübungspunkt und anschließender Phasentrennung):

Die Herstellung einer verdünnten Gewebeklebstofflösung erfolgte wie im Vergleich für die Beispiele 1 bis 37. Der Lösung wurden pro ml 15 Einheiten Faktor XIII zugesetzt, die Lösung auf etwa 15°C gehalten und 5 Vol.% einer Lösung, enthaltend 10 g Pluronic L 61 (siehe Tabelle 2) pro Liter, unter Rühren zugesetzt. Danach wurde die Lösung unter Rühren langsam auf etwa 30°C erwärmt. Die dabei entstandene Emulsion wurde durch scharfe Zentrifugation (ca. 30.000 x g, 30 min, 30°C) in zwei Phasen getrennt.

Die tieferliegende, tensidarme Phase ( = Hauptmenge) wurde nach dieser Behandlung weiterverarbeitet. Dadurch wurden lipophile, schwerlösliche Bestandteile aus der verdünnten Gewebeklebstofflösung entfernt.

Es wurde festgestellt, daß unter den angegebenen Bedingungen der Rest-Tensid-Gehalt der tensidarmen Phase etwa 0,02 g/l beträgt.

Die so erhaltene, gereinigte, verdünnte Gewebeklebstofflösung wurde im Ganzen lyophilisiert, auf einen Wassergehalt von 0,075 (7,5 % Masse) eingestellt und unter $N_2$-Atmosphäre 10 h lang auf 60°C erhitzt. Danach wurde das Lyophilisat zu einer Proteinkonzentration von 50 g/l gelöst, zuerst klarfiltriert und dann sterilfiltriert und zu 12,5 ml in Endbehälter (Glasfläschchen) mit oder ohne magnetische Rührkörper verfüllt und in üblicher Weise tiefgefroren und lyophilisiert.

Nach Rekonstitution mit 5,0 ml Lösungsmittel ($H_2O$ oder wässerige Aprotininlösung) wurden gebrauchsfertige Gewebeklebstofflösungen mit einem Fibrinogengehalt von etwa 80 mg/ml erhalten.

Die benötigten Rekonstitutionszeiten betrugen 13 min nach Methode I und 7 min nach Methode II (Vergleich: 25 bzw. 12 Minuten).

Das Verhältnis Faktor XIII zu Fibrinogen betrug 510 E/g. In ihren übrigen Eigenschaften entsprachen die Präparationen denen der Beispiele 1 bis 39.

Beispiel 42:

Die Arbeitsweise von Beispiel 41 wurde wiederholt, jedoch wurde nach Lösen des hitzebehandelten Lyophilisates der verdünnten Gewebeklebstofflösung noch zusätzlich 0,1 g Triton WR-1339 (siehe Tabelle 2) pro l zugesetzt. Ein Vergleich mit Beispiel 41 (Tabelle 1) zeigt, daß auf diese Weise noch bessere Ergebnisse erzielt werden können.

Das folgende Beispiel 43 zeigt, daß eine Reinigung der verdünnten Gewebeklebstofflösung (Entfernung lipophiler Bestandteile) auch mit einem in $H_2O$ praktisch unlöslichen Tensid, das als Emulsion zugegeben wird, möglich ist. Die nachfolgende Zugabe eines wasserlöslichen Tensides führt ebenfalls zu sehr guten Ergebnissen.

Beispiel 43:

Arbeitsweise wie in Beispiel 42, jedoch wurden anstelle einer Lösung von Pluronic L 61 (siehe Tabelle 2) 5 Vol.% einer 1 Gew.%igen Suspension von Span 80 (siehe Tabelle 2) in $H_2O$ der verdünnten Gewebeklebstofflösung zugesetzt und die Mischung 30 min bei Zimmertemperatur gerührt. Das Tensid Span 80 (siehe Tabelle 2) ist in $H_2O$ nahezu unlöslich.

Nach Trennung der Phasen durch Zentrifugation bei 20ºC wurden der verdünnten Gewebeklebstofflösung noch 0,1 g Triton WR-1339 (siehe Tabelle 2) pro l zugesetzt. Die Weiterverarbeitung erfolgte wie in den Beispielen 41 und 42. Die benötigten Rekonstitutionszeiten betrugen 5,5 min nach Methode I und 3 min nach Methode II (Vergleich: 13 bzw. 7 Minuten).

Die folgenden Beispiele 44 bis 47 zeigen einen günstigen Einfluß von Tensiden auf die Rekonstitutionszeit lyophilisierter Gewebeklebstoffpräparationen auch dann, wenn diese Präparationen nach anderen als dem bisher beschriebenen Verfahren hergestellt werden und ihre Proteinzusammensetzung, insbesondere hinsichtlich des Verhältnisses von Fibronectin zu Fibrinogen und von Albumin zu Fibrinogen, modifiziert wird.

Albumin war in allen bisher bekannten lyophilisierten Gewebeklebstoffpräparationen auf Basis von Fibrinogen und Faktor XIII enthalten, da es die Auflösbarkeit derartiger Präparationen wesentlich verbessert. Durch den erfindungsgemäßen Zusatz einer geringen Menge eines biologisch verträglichen Tensides ist es möglich, derartige Präparationen ohne Albumingehalt herzustellen, wobei diese Präparationen trotzdem sehr leicht rekonstituierbar sind, wie das folgende Beispiel 44 zeigt:

Beispiel 44:

10 l gepooltes humanes Citratplasma wurden bei Zimmertemperatur langsam unter Rühren mit 1500 g pulverförmigem Glycin versetzt, der pH-Wert auf 7,35 eingestellt, und das Gemisch noch eine weitere Stunde bei Raumtemperatur gerührt. Der entstandene Niederschlag wurde durch Zentrifugation abgetrennt und mit 1,5 l einer Lösung, enthaltend 9,0 g NaCl und 2,9 g $Na_3$-Citrat.2 $H_2O$ pro Liter, gelöst.

Die Fällung mit Glycin wurde in analoger Weise, wie oben beschrieben, durch Zugabe von 225 g Glycin wiederholt. Nach Abtrennen des Niederschlages durch Zentrifugation wurde dieser mit der halben Menge $H_2O$ vermischt und gegen eine Lösung, enthaltend 3,6 g NaCl und 1,2 g $Na_3$-Citrat.2 $H_2O$ pro Liter, pH 7,35, bei 4ºC dialysiert, anschließend durch Erwärmen auf 37ºC verflüssigt und mit derselben Lösung auf eine Proteinkonzentration von 40 g/l eingestellt.

Zur Herstellung einer Vergleichspräparation ohne Tensidgehalt wurde ein Teil der Lösung sterilfiltriert und analog den vorhergehenden Beispielen zu je 2,5 ml in Endbehälter verfüllt, tiefgefroren und lyophilisiert.

Zum anderen Teil der Lösung wurde das Tensid WR 1339 (siehe Tabelle 2) in einer Konzentration von 0,1 mg/ml zugesetzt, und die Lösung ebenso sterilfiltriert, verfüllt und lyophilisiert.

Die so erhaltenen Präparationen hatten einen Fibrinogengehalt von 85 Gew.% und waren praktisch frei von Albumin und Fibronectin. Das Verhältnis Faktor XIII zu Fibrinogen betrug 175 Einheiten/g.

Nach Rekonstitution mit 1,0 ml Lösungsmittel ($H_2O$ oder Aprotininlösung) wurden gebrauchsfertige Gewebeklebstofflösungen mit einem Fibrinogengehalt von etwa 95 mg/ml erhalten.

Die elektrische Leitfähigkeit (nach weiterer 10-facher Verdünnung mit $H_2O$) betrug 1,8 mS/cm; die Osmolarität der konzentrierten gebrauchsfertigen Lösung 0,35 Osmol.

Die benötigten Rekonstitutionszeiten (Methode II) betrugen etwa 35 min für die tensidfreie Vergleichspräparation, jedoch nur 3,5 min für die erfindungsgemäße, tensidhaltige Präparation.

Das Beispiel zeigt, daß gerade bei solchen lyophilisierten Präparationen, die an sich besonders schwierig in Lösung zu bringen sind, der erfindungsgemäße Zusatz eines biologisch verträglichen Tensides

9

zu einer enormen und unerwarteten Verbesserung der Auflösbarkeit führt. Dadurch wird es erstmals möglich, auf das relativ teure und nur beschränkt zur Verfügung stehende Humanalbumin als Lösungsvermittler zu verzichten.

Beispiel 45:

20 g eines humanen Plasma-Kryopräzipitates, erhalten entsprechend den Beispielen 1 bis 43, wurden mit 2,0 l einer Lösung, enthaltend 9,0 g NaCl, 2,9 g $Na_3$-Citrat.2 $H_2O$ und 25.000 KIE Aprotinin pro Liter, gelöst und auf 37°C erwärmt. Danach wurden langsam unter Rühren 165 g pulverförmiges Glycin zugesetzt und die Lösung noch eine weitere Stunde unter Abkühlung auf 20°C gerührt. Der entstandene Niederschlag wurde durch Zentrifugation abgetrennt, bei 0 bis 2°C mit der 16-fachen Menge einer Pufferlösung, enthaltend 1,2 g NaCl, 25.000 KIE Aprotinin und 2,4 g $Na_3$-Citrat.2 $H_2O$ pro Liter, pH 7,35, gewaschen und nochmals durch Zentrifugation bei 0 bis 2°C abgetrennt. Danach wurde der Niederschlag mit der oben erwähnten Pufferlösung, die zusätzlich 9,0 g Glycin pro Liter enthielt, gelöst und auf eine Proteinkonzentration von 30 mg/ml eingestellt.

Dieser Lösung wurden sodann 5 Vol.% einer 20 Gew.%igen Humanalbuminlösung, 15 mg gereinigtes, lyophilisiertes Fibronectin und 5 Einheiten Faktor XIII pro ml zugesetzt.

Zur Herstellung einer Vergleichspräparation ohne Tensidgehalt wurde ein Teil der Lösung sterilfiltriert und analog den vorhergehenden Beispielen zu je 2,6 ml in Endbehälter verfüllt, tiefgefroren und lyophilisiert.

Dem anderen Teil der Lösung wurde das Tensid Triton WR 1339 (siehe Tabelle 2) in einer Konzentration von 0,15 mg/ml zugesetzt und die Lösung ebenso sterilfiltriert, verfüllt und lyophilisiert.

Die so erhaltenen Präparationen hatten folgende Zusammensetzung:

Fibrinogen: 41 % Masse der Trockensubstanz

Verhältnis Faktor XIII zu Fibrinogen: 340 E/g

Massenverhältnis Fibronectin zu Fibrinogen: 0,58

Massenverhältnis Albumin zu Fibrinogen: 0,35

Nach Rekonstitution mit 1,0 ml Lösungsmittel ($H_2O$ oder Aprotininlösung) wurde eine gebrauchsfertige Gewebeklebstoff-Lösung mit einem Fibrinogengehalt von 72 mg/ml erhalten.

Die benötigten Rekonstitutionszeiten (Methode II) betrugen für die tensidfreie Vergleichspräparation 15 min, für die erfindungsgemäße tensidhältige Präparation jedoch nur 4 min.

Die elektrische Leitfähigkeit (nach 10-facher Verdünnung mit $H_2O$) betrug 1,3 mS/cm, die Osmolarität der konzentrierten, gebrauchsfertigen Lösung 0,45 Osmol,

Das Beispiel zeigt den günstigen Einfluß des Tensidgehaltes auf die Rekonstitutionszeit von lyophilisierten Gewebeklebstoff-Präparationen mit einem hohen Gehalt an Fibronectin, das ähnlich wie Fibrinogen zu den relativ schwerlöslichen Plasmaproteinen gehört. Der Gehalt an Fibronectin erhöht die Haftfähigkeit des Klebstoffes am Gewebe und dessen wundheilungsfördernde Eigenschaften.

Beispiel 46:

Die Herstellung einer verdünnten Gewebeklebstofflösung mit einem Fibrinogengehalt von 30 mg/ml wurde entsprechend Beispiel 45 wiederholt.

Der Lösung wurden sodann 13 Vol.% einer 20 Gew.%igen Humanalbuminlösung, 3 mg gereinigtes, lyophilisiertes Fibronectin und 5 Einheiten Faktor XIII pro ml zugesetzt. Zur Herstellung einer Vergleichspräparation ohne Tensidgehalt wurde ein Teil der Lösung sterilfiltriert und analog den vorhergehenden Beispielen zu je 14,0 ml in Endbehälter verfüllt, tiefgefroren und lyophilisiert.

Dem anderen Teil der Lösung wurde das Tensid WR 1339 (siehe Tabelle 2) in einer Konzentration von 0,1 mg/ml zugesetzt und die Lösung ebenso sterilfiltriert, verfüllt und lyophilisiert.

Die so erhaltenen Präparationen hatten folgende Zusammensetzung:

Fibrinogen: 40 % Masse der Trockensubstanz

Verhältnis Faktor XIII zu Fibrinogen: 350 E/g

Massenverhältnis Fibronectin zu Fibrinogen: 0,13

Massenverhältnis Albumin zu Fibrinogen: 0,90

Nach Rekonstitution mit 5,0 ml Lösungsmittel wurde eine gebrauchsfertige Gewebeklebstoff-Lösung mit einem Fibrinogengehalt von ca. 72 mg/ml erhalten.

Die benötigten Rekonstitutionszeiten (Methode II) betrugen für die tensidfreie Vergleichspräparation 12 min, für die erfindungsgemäße, tensidhältige Präparation hingegen nur 4 min.

Das Beispiel zeigt den günstigen Einfluß des Tensidgehaltes auf die Rekonstitutionszeit von lyophilisierten Gewebeklebstoffpräparationen mit einem besonders hohen Gesamtproteingehalt (149 mg/ml) bzw. einem besonders hohen Massenverhältnis Albumin zu Fibrinogen.

Der Albumingehalt hat eine stabilisierende Wirkung auf die Präparation, insbesondere wenn diese einem Virusinaktivierungsverfahren (z.B. durch Erhitzen) unterworfen wird.

Beispiel 47:

4,0 l humanes gepooltes Citratplasma wurden mit 100.000 KIE Aprotinin versetzt und danach langsam unter Rühren 760 ml einer bei Zimmertemperatur gesättigten Ammoniumsulfatlösung zugesetzt, der pH-Wert auf 7,0 eingestellt und das Gemisch über Nacht bei 4°C gerührt.

Der entstandene Niederschlag wurde durch Zentrifugation abgetrennt und mit 1,0 l einer Pufferlösung vom pH-Wert 7,4, enthaltend pro Liter 18,0 g NaCl, 14,7 g $Na_3$-Citrat.2 $H_2O$ und 25.000 KIE Aprotinin, gelöst.

Ein geringer Anteil unlöslichen Materials wurde durch Zentrifugieren entfernt und die Fällung mit Ammoniumsulfat in analoger Weise wiederholt.

Der entstandene Niederschlag wurde durch Zentrifugieren abgetrennt, gegen eine Pufferlösung vom pH-Wert 7,35, enthaltend pro Liter 3,6 g NaCl und 1,2 g $Na_3$-Citrat.2 $H_2O$ bei 4°C dialysiert, anschließend durch Erwärmen auf 37°C verflüssigt, mit derselben Pufferlösung auf eine Proteinkonzentration von 40 mg/ml eingestellt und 3 Vol.% einer 20 Gew.%igen Humanalbuminlösung und 4 Einheiten Faktor XIII pro ml zugefügt. Zur Herstellung einer Vergleichspräparation ohne Tensidgehalt wurde ein Teil der Lösung sterilfiltriert und analog den vorhergehenden Beispielen zu je 5,0 ml in Endbehälter verfüllt, tiefgefroren und lyophilisiert.

Zum anderen Teil der Lösung wurde das Tensid Solutol HS 15 (siehe Tabelle 2) in einer Konzentration von 0,12 g/l zugesetzt und die Lösung in gleicher Weise weiterverarbeitet.

Nach Rekonstitution mit 2,0 ml Lösungsmittel ($H_2O$ oder Aprotinin) wurden gebrauchsfertige Gewebeklebstofflösungen mit einem Fibrinogengehalt von 84 mg/ml erhalten.

Die benötigten Rekonstitutionszeiten (Methode II) betrugen etwa 25 min für die tensidfreie Vergleichspräparation, jedoch nur 5 min für die erfindungsgemäße, tensidhältige Präparation.

Im übrigen hatten die Präparationen folgende Zusammensetzung und Eigenschaften:

Fibrinogen: 71 % Masse der Trockensubstanz

Verhältnis Faktor XIII zu Fibrinogen: 265 E/g

Massenverhältnis Fibronectin zu Fibrinogen: 0,095

Massenverhältnis Albumin zu Fibrinogen: 0,18

Elektrische Leitfähigkeit (nach 10-facher Verdünnung mit $H_2O$): 2,0 mS/cm; Osmolarität der konzentrierten, gebrauchsfertigen Lösung: 0,36 Osmol.

Das Beispiel zeigt, daß auch bei dieser geänderten Herstellungsmethode und bei einem relativ niedrigen Albumingehalt (wobei an sich relativ schwer lösliche Präparationen erhalten werden) der erfindungsgemäße Zusatz eines biologisch verträglichen Tensides zu einer wesentlichen Verbesserung der Auflösbarkeit führt.

Sämtliche in den Beispielen 1 bis 47 beschriebenen erfindungsgemäßen Präparationen erwiesen sich als nicht-zytotoxisch und bildeten weiße Fibrin-Clots von erwünschter zäh-elastischer Konsistenz.

Die wesentlichsten Eigenschaften dieser Präparationen (sowie der dazugehörigen Vergleichspräparationen) sind in Tabelle 1 zusammengestellt.

## Tabelle 1

| Beispiel | Tensid | Verhältnis Tensid : Fibrinogen (g/g) | LM | Methode I LZ in min | Methode II LZ in min | Gerinnbares Protein (g/L) | alpha-p (%) | Bemerkungen |
|---|---|---|---|---|---|---|---|---|
| Vergleich für 1-37 | – | – | H$_2$O | 15 | 7 | 81 | 85 | |
| Vergleich für 1-37 | – | – | Apro-tinin* | 15 | 8 | 81 | 86 | |
| 1 | N1 | 0,0038 | H$_2$O | 5 | 2,5 | 79 | 79 | |
| 2 | N1 | 0,0038 | Apro-tinin | 6 | 3 | 80 | 81 | |
| 3 | N1 | 0,0012 | H$_2$O | 8 | 4 | 81 | 81 | |
| 4 | N2 | 0,0125 | H$_2$O | 6 | 3,5 | 80 | 77 | |
| 5 | N2 | 0,0038 | H$_2$O | 7 | 4 | 80 | 83 | |
| 6 | N2 | 0,0013 | H$_2$O | 8 | 4,5 | 80 | 82 | |
| 7 | N3 | 0,0063 | Apro-tinin | 8,5 | 4 | 79 | 75 | |
| 8 | N4 | 0,0123 | H$_2$O | 7 | 3,5 | 81 | 80 | |
| 9 | N5 | 0,013 | H$_2$O | 5 | 2,5 | 80 | 82 | |
| 10 | N6 | 0,013 | H$_2$O | 7 | 3 | 79 | 80 | |
| 11 | N7 | 0,128 | H$_2$O | 5 | 2 | 78 | 65 | |

* Aprotini: wässerige Aprotininlösung mit 3000 KIE/mL

EP 0 345 246 B1

Fortsetzung der Tabelle 1

| Beispiel | Tensid | Verhältnis Tensid : Fibrinogen (g/g) | LM | Methode I LZ in min | Methode II LZ in min | Gerinnbares Protein (g/l) | alpha-p (%) | Bemerkungen |
|---|---|---|---|---|---|---|---|---|
| 12 | N7 | 0,038 | H$_2$O | 5 | 2 | 79 | 75 | |
| 13 | N7 | 0,012 | H$_2$O | 5 | 2 | 81 | 80 | |
| 14 | N7 | 0,0038 | H$_2$O | 6 | 3 | 80 | 79 | |
| 15 | N7 | 0,0038 | Aprotinin | 6 | 3 | 80 | 80 | |
| 16 | N7 | 0,0012 | H$_2$O | 13 | 6 | 81 | 83 | |
| 17 | N5 | 0,0019 | Aprotinin | 6 | 3 | 80 | 78 | |
| | N7 | 0,0019 | | | | | | |
| 18 | N8 | 0,0013 | H$_2$O | 12 | 6 | 79 | 80 | |
| 19 | N11 | 0,0013 | H$_2$O | 10 | 5 | 80 | 82 | |
| 20 | N12 | 0,013 | H$_2$O | 10 | 4,5 | 80 | 81 | |
| 21 | N7 | 0,013 | H$_2$O | 4,5 | 2 | 80 | 82 | |
| | N9 | 0,0004 | | | | | | |
| 22 | N13 | 0,0038 | H$_2$O | 8 | 3,5 | 79 | 83 | |
| 23 | N17 | 0,0013 | H$_2$O | 8 | 4 | 81 | 83 | N17 enthält ein Antioxidans |

EP 0 345 246 B1

Fortsetzung der Tabelle 1

| Beispiel | Tensid | Verhältnis Tensid : Fibrinogen (g/g) | LM | Methode I LZ in min | Methode II LZ in min | Gerinnbares Protein (g/l) | alpha-p (%) | Bemerkungen |
|---|---|---|---|---|---|---|---|---|
| 24 | Z1 | 0,013 | $H_2O$ | 7 | 3,5 | 80 | 77 | |
| 25 | Z1 | 0,013 | Apro-tinin | 7 | 3,5 | 81 | 79 | |
| 26 | Z2 | 0,004 | $H_2O$ | 10 | 5 | 81 | 80 | |
| 27 | Z3 | 0,004 | $H_2O$ | 9 | 4 | 81 | 77 | |
| 28 | Z4 | 0,004 | $H_2O$ | 10 | 4,5 | 80 | 79 | |
| 29 | Z5 | 0,004 | $H_2O$ | 8 | 4 | 79 | 82 | |
| 30 | Z6 | 0,013 | $H_2O$ | 6 | 3 | 81 | 80 | |
| 31 | A1 | 0,013 | $H_2O$ | 8 | 3,5 | 80 | 78 | |
| 32 | A1 | 0,013 | Apro-tinin | 7,5 | 3,5 | 80 | 80 | |
| 33 | A2 | 0,004 | $H_2O$ | 10 | 5 | 79 | 81 | |
| 34 | K1 | 0,004 | $H_2O$ | 8 | 3,5 | 80 | 77 | |
| 35 | K1 | 0,004 | Apro-tinin | 7,5 | 3,5 | 80 | 82 | |
| 36 | K2 | 0,004 | $H_2O$ | 8 | 4 | 80 | 79 | |
| 37 | A2 | 0,013 | $H_2O$ | 7,5 | 3,5 | 81 | 80 | |
| | K1 | 0,006 | | | | | | |

EP 0 345 246 B1

Fortsetzung der Tabelle 1

| Beispiel | Tensid | Verhältnis Tensid : Fibrinogen (g/g) | LM | Methode I LZ in min | Methode II LZ in min | Gerinnbares Protein (g/l) | alpha-p (%) | Bemerkun-gen |
|---|---|---|---|---|---|---|---|---|
| Vergleich für 38 | - | - | H$_2$O | 20 | 10 | 80 | 95 | |
| Vergleich für 38 a | - | - | Apro-tinin | 20 | 9 | 81 | 93 | |
| 38 | N5 | 0,003 | H$_2$O | 7 | 3 | 81 | 91 | |
| 38 a | N5 | 0,003 | Apro-tinin | 7 | 4 | 80 | 92 | |
| Vergleich für 39 | - | - | Apro-tinin | 25 | 12 | 78 | 89 | |
| 39 | N7 | 0,003 | Apro-tinin | 8 | 4 | 80 | 91 | |
| Vergleich für 40 | - | - | Apro-tinin | - | 25 | 81 | 85 | Mit Anti-biotikum |
| 40 | N7 | 0,003 | Apro-tinin | - | 4 | 80 | 86 | Mit Anti-biotikum |
| Vergleich für 41,42,43 | - | - | - | 25 | 12 | 78 | 89 | |
| 41 | N19 | 0,0006 | Apro-tinin | 13 | 7 | 79 | 90 | |

EP 0 345 246 B1

Fortsetzung der Tabelle 1

| Beispiel | Tensid | Verhältnis Tensid : Fibrinogen (g/g) | LM | Methode I LZ in min | Methode II LZ in min | Gerinnbares Protein (g/l) | alpha-p (%) | Bemerkungen |
|---|---|---|---|---|---|---|---|---|
| 42 | N19 | 0,0006 | Apro- | 5 | 2,5 | 79 | 93 | |
| | N7 | 0,003 | tinin | | | | | |
| 43 | N20 | n.b. | $H_2O$ | 5,5 | 3 | 80 | 92 | |
| | N7 | 0,003 | | | | | | |
| Vergleich für 44 | - | - | Apro- tinin | - | 35 | 94 | 87 | |
| 44 | N7 | 0,0026 | Apro- tinin | - | 3,5 | 95 | 85 | |
| Vergleich für 45 | - | - | $H_2O$ | - | 15 | 72 | 92 | |
| 45 | N7 | 0,0054 | $H_2O$ | - | 4 | 72 | 90 | |
| Vergleich für 46 | - | - | $H_2O$ | - | 12 | 72 | 95 | |
| 46 | N7 | 0,0039 | $H_2O$ | - | 4 | 73 | 95 | |
| Vergleich für 47 | - | - | Apro- tinin | - | 25 | 84 | 88 | |
| 47 | N1 | 0,0036 | Apro- tinin | - | 5 | 84 | 87 | |

LM = Lösungsmittel

LZ = Lösungszeit (Rekonstitutionszeit)

alpha-p = Vernetzung der Fibrin-alpha-Ketten (Fb-alpha-Polymer)

n.b. = nicht bestimmt

EP 0 345 246 B1

## Tabelle 2

N: nichtionisch    A: anionisch    K: kationisch    Z: zwitterionisch

| Code des Tensids | chemische Bezeichnung | Handelsbezeichnung |
|---|---|---|
| N1 | Polyäthylenglykol-660-12-hydroxystearat | Solutol HS 15 |
| N2 | Polyoxyäthylen-(ca. 40)-stearoylester | Myrj 52 |
| N3 | 2,4,7,9-Tetramethyl-5-decin-4,7-diol | Surfynol |
| N4 | Polyäthylenglykol(20)-Sorbitan-monooleat | Tween 80 |
| N5 | Polyoxyäthylen-polyoxypropylen-Blockpolymer | Pluronic F 108 |
| N6 | Octyl-ß-D-glucopyranosid | - |
| N7 | Formaldehydpolymer des Octylphenolpolyoxyäthylenäthers | Triton WR 1339 |
| N8 | Dodecyldimethylaminoxid | - |
| N9 | Sorbitan-monolaurat | Span 20 |
| N10 | Octylphenolpolyäthylenglykol(40)äther | Triton X-405 |
| N11 | Sucrose-palmitat-stearat (SPS 15) | - |
| N12 | N-D-Gluco-N-methyldecanamid (Mega 10) | - |
| N13 | Octylphenolpolyäthylenglykol(7-8)äther | Triton X-114 |
| N14 | Octylphenolpolyäthylenglykol(12-13)äther | Triton X-102 |
| N15 | Octylphenolpolyäthylenglykol(30)äther | Triton X-305 |
| N16 | Polyoxyäthylen(10)hexadecyläther | Brij 56 |
| N17 | Polyoxyäthylen(20)hexadecyläther | Brij 58 |
| N18 | Polyoxyäthylen(23)dodecyläther | Brij 35 |

Fortsetzung der Tabelle 2

| Code des Tensids | chemische Bezeichnung | Handelsbezeichnung |
|---|---|---|
| N19 | Polyoxyäthylen-polyoxypropylen-Blockpolymer | Pluronic L 61 |
| N20 | Sorbitanmonooleoat | Span 80 |
| A1 | Dioctylsulfosuccinat | – |
| A2 | Na-Desoxycholat | – |
| K1 | Benzyldimethyl-2-hydroxyäthyl-ammoniumchlorid | – |
| K2 | Benzyltrimethylammoniumchlorid | – |
| K3 | Cetylpyridiniumchlorid | – |
| Z1 | 3-(3-Cholamidopropyl)-dimethylammonio-1-propansulfonat (Chaps) | – |
| Z2 | Lecithin | – |
| Z3 | N-Dodecyl-N',N-dimethylammonio-3-propansulfonat (Sulfobetain SB 12) | – |
| Z4 | N-Lauryl-ß-iminodipropionsäure | – |
| Z5 | Alkylbetain | Empigen BB |
| Z6 | N-Lauroylsarkosin | Sarkosyl NL-35 |

**Patentansprüche**

1. Gewebeklebstoff zum nahtlosen bzw. nahtunterstützenden Verbinden von menschlichen oder tierischen Gewebe- oder Organteilen, zur Wundversiegelung, Blutstillung und Förderung der Wundheilung in

18

lyophilisierter Form mit einem Gehalt an Fibrinogen von mindestens 0,25 (25 % Masse) und einem Gehalt an Faktor XIII, wobei das Mengenverhältnis des Faktors XIII zu Fibrinogen, ausgedrückt in Einheiten des Faktors XIII pro Gramm Fibrinogen, mindestens 150 beträgt, dadurch gekennzeichnet, daß neben Fibrinogen und gegebenenfalls weiteren Proteinen sowie Hilfs- oder Zusatzstoffen mindestens ein Tensid aus den Gruppen der nichtionischen, kationischen, anionischen oder zwitterionischen Tenside in einer Menge von 0,0003 bis 0,15 (0,03 bis 15 % Masse), vorzugsweise 0,001 bis 0,01 (0,1 bis 1,0 % Masse), bezogen auf den Fibrinogen-Gehalt, enthält.

2. Gewebeklebstoff nach Anspruch 1, dadurch gekennzeichnet, daß er ein Tensid, ausgewählt aus den Gruppen der Polyätheralkohole, umfassend Polyoxyäthylen(23)dodecyläther, Polyoxyäthylen(10)-hexadecyläther, Polyoxyäthylen(20)hexadecyläther, Octylphenolpolyäthylenglykol(30)äther, Octylphenolpolyäthylenglykol(12-13)äther, Octylphenolpolyäthylenglykol(7-8)äther, Octylphenolpolyäthylenglykol(40)äther und Octylphenolpolyäthylenglykolätherformaldehyd-polymere, der Polyätherester, wie Polyäthylenglykol-660-12-hydroxystearat, Polyoxyäthylen-stearoylester, oder der Polyoxyäthylen-polyoxypropylen-Blockpolymeren enthält.

3. Gewebeklebstoff nach Anspruch 1, dadurch gekennzeichnet, daß er ein Tensid, ausgewählt aus der Gruppe der Zuckerester, wie Sucrose-palmitat-stearat, der Polyalkoholanhydridester, wie Sorbitanmonolaurat oder Sorbitanmonooleoat, der Glycoside, wie Octyl-$\beta$-D-glucopyranosid, der Alkinole, wie 2,4,7,9-Tetramethyl-5-decin-4,7-diol, der Aminoxide, wie Dodecyldimethylaminoxid, oder der Hydroxyalkylamide, enthält.

4. Gewebeklebstoff nach Anspruch 1, dadurch gekennzeichnet, daß er ein Tensid, ausgewählt aus der Gruppe der Sulfosuccinate, wie Dioctylsulfosuccinat, oder der Alkalisalze der Gallensäuren, wie Na-Desoxycholat, enthält.

5. Gewebeklebstoff nach Anspruch 1, dadurch gekennzeichnet, daß er ein Tensid, ausgewählt aus der Gruppe der substituierten Ammoniumsalze, wie Benzyldimethyl-2-hydroxyäthyl-ammoniumchlorid oder Benzyltrimethylammoniumchlorid, oder der Alkylpyridiniumsalze, wie Cetylpyridiniumchlorid, enthält.

6. Gewebeklebstoff nach Anspruch 1, dadurch gekennzeichnet, daß er ein Tensid, ausgewählt aus der Gruppe der Phosphatide, wie Lecithin, oder mindestens ein Tensid aus der Gruppe der Sulfobetaine, wie N-Dodecyl-N'N-dimethylammonio-3-propansulfonat, der zwitterionischen Gallensäurenderivate, wie 3-(3-Cholamidopropyl)dimethylamino-1-propansulfonat, der Alkylbetaine, der Sarkosine, wie N-Lauroylsarkosin, oder der Iminodipropionsäuren, wie N-Lauryl-$\beta$-iminodipropionsäure, enthält.

7. Gewebeklebstoff nach einem oder mehreren der Ansprüche 1 bis 6, dadurch gekennzeichnet, daß er zusätzlich ein Antioxidans enthält.

8. Gewebeklebstoff nach einem oder mehreren der Ansprüche 1 bis 7, dadurch gekennzeichnet, daß er zusätzlich Substanzen mit antimikrobieller Wirkung enthält.

9. Verwendung einer lyophilisierten Zusammensetzung auf Basis von menschlichen oder tierischen Proteinen mit einem Gehalt an Fibrinogen von mindestens 0,25 (25 % Masse) der Trockensubstanz, einem Gehalt an Faktor XIII in einer Menge von mindestens 150 Einheiten pro Gramm Fibrinogen und einem Gehalt an mindestens einem biologisch verträglichen Tensid nach einem der vorhergehenden Ansprüche, wobei der Gesamttensidgehalt 0,0003 bis 0,15 (0,03 bis 15 % Masse) des Fibrinogengehaltes beträgt, gegebenenfalls mit einem Gehalt an Fibronectin bis zu 0,65 (65 % Masse) des Fibrinogengehaltes und gegebenenfalls mit einem Gehalt an Albumin bis zu 1,1 (110 % Masse) des Fibrinogengehaltes, zur Herstellung einer gebrauchsfertigen Gewebeklebstofflösung in einer Konzentration von mindestens 70 mg Fibrinogen/ml zum nahtlosen bzw. nahtunterstützenden Verbinden von menschlichen oder tierischen Gewebe- oder Organteilen, zur Wundversiegelung, Blutstillung, Förderung der Wundheilung.

## Claims

1. A tissue adhesive for the suture-less or suture-supporting connection of human or animal tissue or organ parts, for the sealing of wounds, the stoppage of bleeding and the acceleration of wound healing, in lyophilised form having a content of fibrinogen of at least 0.25 (25 % by mass) and a content of factor XIII, the volume ratio of factor XIII to fibrinogen, expressed in units of factor XIII per gramme of fbirinogen, being at least 150, characterized in that, in addition to fibrinogen and, if desired, further proteins as well as auxiliary substances or additives, it contains at least one tenside from the group of non-ionic, cationic, anionic or zwitterionique tensides in an amount of from 0.0003 to 0.15 (0.03 to 15 % by mass), preferably from 0.001 to 0.01 (0.1 to 1.0 % by mass), based on the fibrinogen content.

2. A tissue adhesive according to claim 1, characterized in that it contains a tenside selected from the groups of polyether alcohols comprising polyoxyethylene(23)dodecyl ether, polyoxyethylene(10)-hexadecyl ether, polyoxyethylene(20)hexadecyl ether, octylphenolpolyethyleneglycol(30) ether, octylphenolpolyethyleneglycol(12-13) ether, octylphenolpolyethyleneglycol(7-8) ether, octylphenolpolyethyleneglycol(40)ether, and octylphenolpolyethyleneglycolether formaldehyde polymers, of polyether esters, such as polyethyleneglycol-660-12-hydroxystearate, polyoxyethylene stearoyl ester, or polyoxyethylene polyoxypropylene block polymers.

3. A tissue adhesive according to claim 1, characterized in that it contains a tenside selected from the group of sugar esters, such as sucrose palmitate stearate, polyalcohol anhydride esters, such as sorbitane monolaurate or sorbitane monooleate, glycosides, such as octyl-$\beta$-D-glucopyranoside, alcinols, such as 2,4,7,9-tetramethyl-5-decin-4,7-diol, aminoxides, such as dodecyldimethylaminoxide, or hydroxyalkylamides.

4. A tissue adhesive according to claim 1, characterized in that it contains a tenside selected from the group of sulfosuccinates, such as dioctyl sulfosuccinate, or alkali salts of bile acids, such as Na-desoxycholate.

5. A tissue adhesive according to claim 1, characterized in that it contains a tenside selected from the group of substituted ammonium salts, such as benzyldimethyl-2-hydroxyethyl ammonium chloride or benzyltrimethyl ammonium chloride, or alkyl pyridinium salts, such as cetyl pyridinium chloride.

6. A tissue adhesive according to claim 1, characterized in that it contains a tenside selected from the group of phosphatides, such as lecithin, or at least a tenside from the group of sulfobetains, such as N-dodecyl-N'N-dimethylammonio-3-propane sulfonate, of zwitterionic bile acid derivatives, such as 3-(3-cholamidopropyl)-dimethylamino-1-propane sulfonate, of alkyl betains, of sarcosins, such as N-lauroyl sarcosin, or of iminodipropionic acids, such as N-lauryl-$\beta$-iminodipropionic acid.

7. A tissue adhesive according to one or several of claims 1 to 6, characterized in that it additionally contains an antioxidant.

8. A tissue adhesive according to one or several of claims 1 to 7, characterized in that it additionally contains substances having antimicrobial activity.

9. The use of a lyophilised composition based on human or animal proteins and having a content of fibrinogen of at least 0.25 (25 % by mass) of dry matter, a content of factor XIII in an amount of at least 150 units per gramme of fibrinogen and a content of at least one biologically safe tenside according to one of the preceding claims, the overall tenside content being 0.0003 to 0.15 (0.03 to 15 % by mass) of the fibrinogen content, if desired having a content of fibronectin up to 0.65 (65 % by mass) of the fibrinogen content and, if desired, having a content of albumin up to 1.1 (110 % by mass) of the fibrinogen content, for the preparation of a ready-for-use tissue adhesive solution at a concentration of at least 70 mg fibrinogen/ml for the suture-less or suture-supporting connection of human or animal tissue or organ parts, for the sealing of wounds, the stoppage of bleeding and the acceleration of wound healing.

**Revendications**

1. Colle pour tissus destiné à réunir des parties de tissus ou d'organes humaines ou animales sans sutures ou en supportant des sutures, à sceller des plaies, à arrêter le sang et à favoriser la cicatrisation, en forme lyophilisée ayant une teneur en fibrinogène d'au moins 0,25 (25 % en masse) et une teneur en facteur XIII, la proportion volumétrique du facteur XIII au fibrinogène, exprimée en unités du facteur XIII par gramme de fibrinogène, étant au moins 150, caractérisée en ce qu'elle contient, en plus de fibrinogène et, le cas écheant, d'autres protéines ainsi que de matières auxiliaires ou additionnelles, au moins un tensio-actif des groupes des tensio-actifs non-ioniques, cationiques, anioniques ou zwitterioniques en une quantité de 0,0003 à 0,15 (0,03 à 15 % en masse), de préférence 0,001 à 0,01 (0,1 à 1,0 % en masse), par rapport à la teneur en fibrinogène.

2. Colle pour tissus selon la revendication 1, caractérisée en ce qu'elle contient un tensio-actif choisi parmi les groupes des alcools de polyéther comprenant polyoxyéthylène(23)dodecyléther, polyoxyéthylène(10)hexadecyléther, polyoxyéthylène(20)hexadecyléther, octylphenolpolyéthylèneglycol-(30)éther, octylphenolpolyéthylèneglycol(12-13)éther, octylphenolpolyéthylèneglycol(7-8)éther, octylphenolpolyéthylèneglycol(40)éther et des polymères d'octylphenolpolyéthylèneglycolétherformaldéhyde, des esters de polyéther, tel que polyethylèneglycol-660-12-hydroxystéarate, des esters stéaroyliques de polyoxyethylène ou des polymères en masse de polyoxyéthylène-polyoxypropylène.

3. Colle pour tissus selon la revendication 1, caractérisée en ce qu'elle contient un tensio-actif choisi parmi le groupe des esters de sucre, tel que sucrose-palmitate-stéarate, des esters d'anhydrides de polyalcool, tel que sorbitanne-monolaureate ou sorbitanne-monooléate, des glycosides, tel que octyl-$\beta$-D-glucopyranoside, des alcinoles, tel que 2,4,7,9-tetramethyl-5-decin-4,7-diol, des aminoxides, tel que dodecyldimethylaminoxide, ou des hydroxyalkylamides.

4. Colle pour tissus selon la revendication 1, caractérisée en ce qu'elle contient un tensio-actif choisi parmi le groupe des sulfosuccinates, tel que dioctylsulfosuccinate, ou des sels alcalins des acides biliaires, tel que Na-desoxycholate.

5. Colle pour tissus selon la revendication 1, caractérisée en ce qu'elle contient un tensio-actif choisi parmi le groupe des sel d'ammonium substitués, tel que le chlorure d'ammonium de benzyldimethyl-2-hydroxyethyl ou le chlorure d'ammonium de benzyltrimethyl, ou des sels d'alkylpyridinium, tel que le chlorure de cetylpyridinium.

6. Colle pour tissus selon la revendication 1, caractérisée en ce qu'elle contient un tensio-actif choisi parmi le groupe des phosphatides, tel que le lecithin, ou au moins un tensioactif du le groupe des sulfobetaïnes, tel que N-dodecyl-N'N-dimethylammonio-3-propanesulfonate, des dérivés zwitterioniques d'acides biliaires, tel que 3-(3-cholamidopropyl)-dimethylamino-1-propanesulfonate, des alkylbetaïnes, des sarcosines, tel que N-lauroylsarcosine, ou des acides iminodipropioniques, tel que l'acide N-lauryl-$\beta$-iminodipropionique.

7. Colle pour tissus selon une ou plusieurs des revendications 1 à 6, caractérisée en ce qu'elle contient en plus un agent antioxidant.

8. Colle pour tissus selon une ou plusieurs des revendications 1 à 7, caractérisée en ce qu'elle contient en plus des matières ayant une activité antimicrobielle.

9. L'utilisation d'une composition lyophilisée sur base des protéines humaines ou animales ayant une teneur en fibrinogène d'au moins 0,25 (25 % en masse) de la matière sèche, une teneur en facteur XIII en une quantité d'au moins 150 unités par gramme de fibrinogène, et une teneur en au moins un tensio-actif biologiquement compatible selon une des revendications précédentes, la teneur totale en tensio-actif étant 0,0003 à 0,15 (0,03 à 15 % en masse) de la teneur en fibrinogène, le cas échéant, une teneur en fibronectin jusqu'à 0,65 (65 % en masse) de la teneur en fibrinogène et, le cas échéant, une teneur en albumine jusqu'à 1,1 (110 % en masse) de la teneur en fibrinogène, pour la préparation d'une solution de colle pour tissus prête à l'emploi, en une concentration d'au moins 70 mg fibrinogène/ml, à réunir des parties de tissus ou d'organes humaines ou animales sans sutures ou pour supportant des sutures, à sceller des plaies, à arrêter le sang et à favoriser la cicatrisation.